# EUROPÄISCHE PATENTANMELDUNG

(11) **EP 3 964 390 A1**
(43) Veröffentlichungstag der Anmeldung: **09.03.2022**
(21) Anmeldenummer: 21192408.9
(22) Anmeldetag: 20.08.2021
(51) Int. Cl.: B60N 2/90, A61B 5/00

(54) **FAHRZEUGSITZ MIT FLUIDKAMMEREINHEIT**

(30) Priorität: 08.09.2020 DE 102020123395
(71) Anmelder: GRAMMER AG, 92289 Ursensollen (DE)
(72) Erfinder: SCHNEIDER, Florian, 92224 Amberg (DE); FROHRIEP, Susanne, 92224 Amberg (DE); SCHUSTJEW, Sergej, 92237 Sulzbach-Rosenberg (DE)
(74) Vertreter: Hannke, Christian

(57) **Zusammenfassung**

Die Erfindung betrifft einen Fahrzeugsitz mit einem Sitzoberteil zum Aufnehmen einer Person, welches ein Sitzplattenteil und ein Sitzpolsterteil umfasst, wobei das Sitzpolsterteil bezogen auf eine Höhenachse des Fahrzeugsitzes oberhalb des Sitzplattenteils angeordnet ist und wobei das Sitzplattenteil und das Sitzpolsterteil bezogen auf die Höhenachse zumindest teilweise, einen dazwischenliegenden ersten Überlappungsbereich ausbildend, überlappend angeordnet sind, wobei eine Fluidkammereinheit mit mindestens zwei Fluidkammern zumindest teilweise innerhalb des ersten Überlappungsbereichs zwischen dem Sitzplattenteil und dem Sitzpolsterteil angeordnet ist, wobei eine Steuereinheit vorgesehen ist, dazu ausgebildet, ein Fluid in die mindestens zwei Fluidkammern einzubringen und zu entnehmen, sodass eine Ausdehnung in der Höhenachse (Z) der mindestens zwei Fluidkammern (10) steuerbar ist.

## Beschreibung

Die Erfindung betrifft einen Fahrzeugsitz mit einem Sitzoberteil zum Aufnehmen einer Person, welches ein Sitzplattenteil und ein Sitzpolsterteil umfasst, wobei das Sitzpolsterteil bezogen auf eine Höhenachse des Fahrzeugsitzes oberhalb des Sitzplattenteils angeordnet ist und wobei das Sitzplattenteil und das Sitzpolsterteil bezogen auf die Höhenachse des Fahrzeugsitzes zumindest teilweise, einen dazwischenliegenden ersten Überlappungsbereich ausbildend, überlappend angeordnet sind, gemäß dem Oberbegriff des Anspruches 1. Ferner betrifft die Erfindung ein System mit einem Fahrzeugsitz und ein Verfahren zum Betreiben des Systems.

In Kraftfahrzeugen, insbesondere in Nutzfahrzeugen, wie Traktoren oder LKW, ist es aufgrund der unter Umständen langen Fahrtzeiten wichtig, dem Fahrer einen höchstmöglichen Sitzkomfort zu bieten. Aus dem Stand der Technik sind derartige Fahrzeugsitze bekannt, die es ermöglichen, die Sitzposition eines Fahrzeugführers nach dessen Wünschen bezüglich der Ergonomie und dem Fahrempfinden einzustellen. Die entsprechenden Verstellmöglichkeiten, wie beispielsweise die Neigung der Sitzhöhe, die Position und Ausrichtung von einzelnen Komponenten des Sitzes wie Rückenlehne, Kopfteil, Sitzpolsterteil oder aber auch Einstellung einer Feder- und/oder Dämpfeinrichtung werden dabei meist vor dem Fahrtbeginn nacheinander eingestellt und während einer Fahrt nicht mehr oder kaum mehr verändert.

Ein Fahrer, der sich besonders bei langen Fahrten lange - ohne Bewegung - in der gleichen Sitzposition befindet, kann durch eine monotone und einseitige Sitzhaltung, Schmerzen durch Überbelastung von Körperteilen, insbesondere der Muskeln und Bandscheiben, erfahren. Weiterhin kann ein statischer Diskomfort auftreten. Neben den körperlichen bzw. gesundheitlichen Beeinträchtigungen können lange Fahrten zu Ermüdungserscheinungen führen, die wiederrum eine Verminderung der Aufmerksamkeit und der Reaktionszeit nach sich ziehen können, wodurch das Unfallrisiko gerade auf langen Strecken ohne Pausen deutlich erhöht ist.

Es ist daher die Aufgabe der Erfindung, einen Fahrzeugsitz zur Verfügung zu stellen, der die genannten Nachteile des Standes der Technik behebt und körperlichen Beeinträchtigungen und Ermüdungserscheinungen, insbesondere bei langen Fahrzeiten, vorbeugt.

Diese Aufgabe wird gelöst von einem Fahrzeugsitz mit einem Sitzoberteil zum Aufnehmen einer Person, welches ein Sitzplattenteil und ein Sitzpolsterteil umfasst, wobei das Sitzpolsterteil, bezogen auf eine Höhenachse des Fahrzeugsitzes, die oberhalb des Sitzplattenteils angeordnet ist und wobei das Sitzplattenteil und das Sitzpolsterteil bezogen auf die Höhenachse des Fahrzeugsitzes zumindest teilweise, einen dazwischenliegenden ersten Überlappungsbereich ausbildend, überlappend angeordnet sind, wobei eine Fluidkammereinheit mit mindestens zwei Fluidkammern zumindest teilweise innerhalb des ersten Überlappungsbereichs zwischen dem Sitzplattenteil und dem Sitzpolsterteil angeordnet ist, wobei eine Steuereinheit vorgesehen ist, dazu ausgebildet, ein Fluid in die mindestens zwei Fluidkammern einzubringen und zu entnehmen, sodass eine Ausdehnung in der Höhenachse der mindestens zwei Fluidkammern steuerbar ist.

Erfindungsgemäß wird unter einem Sitzoberteil der Teil eines Fahrzeugsitzes verstanden, auf welchem eine Person, bevorzugt der Fahrer des Fahrzeugs, Platz nimmt. Das Sitzoberteil umfasst dabei das Sitzplattenteil und das Sitzpolsterteil, wobei eine Person bevorzugt auf dem Sitzpolsterteil ruht. Das Sitzpolsterteil ist bevorzugt aus einem flexiblen, reversibel verformbaren Material, bevorzugt einem Hartschaum, hergestellt. Bevorzugt ist das Sitzpolsterteil dabei kraftschlüssig, formschlüssig oder stoffschlüssig mit einer Oberseite des Sitzplattenteils verbunden. Das Sitzplattenteil ist weiter bevorzugt verbunden mit einem Sitzbasisteil angeordnet, wobei das Sitzbasisteil bevorzugt kraftschlüssig, formschlüssig oder stoffschlüssig mit einer Karosserie des Fahrzeuges verbunden sein kann oder das Sitzbasisteil kann über eine bekannte Federungs- und/oder Dämpfungseinrichtung mit der Fahrzeugkarosserie verbunden sein. Bevorzugt weist der erfindungsgemäße Fahrzeugsitz ebenfalls ein Rückenlehnenteil auf, welches bevorzugt mit dem Sitzbasisteil verbunden ist und ein Rückenplattenteil und ein Rückenpolsterteil umfasst. Der erfindungsgemäße Fahrzeugsitz ist bevorzugt in einem Kraftfahrzeug, besonders bevorzugt in einem Nutzfahrzeug, wie einem Nutzfahrzeug zum Personentransport (z.B. Bus), einem Nutzfahrzeug zur Lastenbeförderung (z.B. Lastkraftwagen) oder anderen Nutzfahrzeugen, angeordnet.

Der Fahrzeugsitz erstreckt sich entlang einer Höhenachse, einer Breitenachse und einer Längenachse, wobei den jeweiligen Achsen jeweils zwei Richtungen zuzuordnen sind. So umfasst die Höhenachse die Richtungen nach oben und nach unten. Die Breitenachse umfasst die Richtungen nach rechts und nach links. Die Längenachse umfasst die Richtungen nach vorne und nach hinten.

Durch die erfindungsgemäße Fluidkammereinheit mit mindestens zwei Fluidkammern, welche durch eine Steuereinheit steuerbar mit einem Fluid befüllbar ist und das Fluid auch wieder entnehmbar ist, können Mikrobewegungen in dem Sitzpolsterteil durch Kraftübertragung von der Fluidkammereinheit auf das Sitzpolsterteil erzeugt werden, welche eine Person, die auf dem Fahrzeugsitz sitzt, mobilisieren, da die Mikrobewegungen von dem Sitzpolsterteil auf die Person übertragen werden. Dabei kommt es zu einer reversiblen Verformung des Sitzpolsterteils. Die Mikrobewegungen können dabei, insbesondere auf langen Fahrten, die Bandscheibenelastizität fördern und so Bandscheibendefekte vorbeugen, den statischen Diskomfort reduzieren oder auch verhindern, lange statische Muskelspannung vermeiden und die Muskelermüdung reduzieren sowie die Aufmerksamkeit und Reaktionszeit der auf dem Fahrzeugsitz sitzenden Person verbessern.

Die Steuereinheit ist dabei in oder an dem Fahrzeugsitz angeordnet oder befindet sich an einer beliebigen Position innerhalb des Fahrzeuges, in dem der Fahrzeugsitz angeordnet ist. Es ist auch denkbar, dass die erfindungsgemäße Steuereinheit eine bereits vorhandene Steuereinheit des Fahrzeuges ist, die beispielsweise mittels einer geeigneten Software die Aufgaben übernehmen kann.

Bevorzugt handelt es sich bei dem Fluid um ein Gas oder einer Flüssigkeit. Bevorzugt handelt es sich bei dem Gas um Luft. Weiter bevorzugt handelt es sich bei der Flüssigkeit um Wasser. Dabei ist es denkbar, dass die Fluidkammereinheit hinsichtlich Material und Ausgestaltung entsprechend an das verwendete Fluid angepasst ist. Unter einem Einbringen des Fluids in die Fluidkammereinheit bzw. die mindestens zwei Fluidkammern wird erfindungsgemäß ein aktives Einbringen verstanden, wobei darunter wiederrum ein Einbringen unter Druck (z.B. Druckluft) verstanden wird. Dies ist beispielsweise mit bekannten Pumpen (Flüssigkeit) oder Verdichtern (Gas) möglich. Unter einem Entnehmen des Fluids kann entweder ein aktives Entnehmen oder ein passives Entnehmen verstanden werden. Dabei wird bei einem aktiven Entnehmen das Fluid beispielsweise mittels Anlegen eines Unterdrucks entnommen. Ein passives Entnehmen kann beispielsweise über das Gewicht einer Person, die auf dem Sitzpolsterteil sitzt, initiiert werden, wobei hierzu die Steuereinheit bevorzugt ein geeignet angeordnetes Ventil gesteuert öffnet, über welches das Fluid abfließen bzw. entnommen werden kann, da das Gewicht, der auf dem Fahrzeugsitz sitzenden Person, einen Druck durch die Gewichtskraft auf die Fluidkammereinheit bzw. die mindestens zwei Fluidkammern ausübt. Bevorzugt sind eine zu entnehmende Fluidmenge und eine Entnahmerate (Fluidförderrate) durch die Steuereinheit, beispielsweise über die Öffnungsdauer und -weite eines Ventils, einstellbar.

Gemäß einer bevorzugten Ausführungsform sind die mindestens zwei Fluidkammern jeweils dazu ausgebildet, das Fluid aufzunehmen. Bevorzugt ist eine zumindest signaltechnisch mit der Steuereinheit verbundene Fluidfördereinrichtung vorgesehen, die zumindest fluidisch mit jeder der mindestens zwei Fluidkammern unabhängig verbunden ist und dazu ausgebildet ist, das Fluid in und/oder aus jeder der mindestens zwei Fluidkammern zu transportieren, wobei das Fluid in jede der mindestens zwei Fluidkammern individuell einbringbar und aus jeder der mindestens zwei Fluidkammern individuell entnehmbar ist. Bei der Fluidfördereinrichtung handelt es sich bevorzugt um eine Pumpe oder einen Verdichter. Bevorzugt ist die Fluidfördereinrichtung in oder an dem Fahrzeugsitz angeordnet, wobei es auch denkbar ist, dass die Fluidfördereinrichtung benachbart zu dem Fahrzeugsitz oder irgendwo in dem Fahrzeug, in dem sich der Fahrzeugsitz befindet, angeordnet ist. Durch die Fluidfördereinrichtung, welche mit den mindestens zwei Fluidkammern der Fluidkammereinheit verbunden ist, ist das Fluid steuerbar durch die Steuereinheit individuell in die mindestens zwei Fluidkammern einbringbar und/oder entnehmbar.

Gemäß einer denkbaren Ausführungsform sind die mindestens zwei Fluidkammern durch Einbringen mindestens einer ersten definierten Fluidmenge jeweils unabhängig voneinander von einem ersten Zustand in mindestens einen zweiten Zustand und durch Entnehmen der mindestens einen ersten definierten Fluidmenge jeweils unabhängig voneinander von dem mindestens einen zweiten Zustand in den ersten Zustand überführbar, wobei die mindestens zwei Fluidkammern in dem ersten Zustand eine erste Ausdehnung in der Höhenachse des Fahrzeugsitzes und in dem mindestens einen zweiten Zustand mindestens eine zweite Ausdehnung in der Höhenachse des Fahrzeugsitzes aufweisen, wobei sich die erste und die mindestens eine zweite Ausdehnung in der Höhenachse unterscheiden. Es sind eine Vielzahl von zweiten Zuständen möglich, je nach eingebrachter bzw. entnommener Fluidmenge. Demnach ist es denkbar, dass der erste Zustand einen Zustand darstellt, in welchem kein bzw. im Wesentlichen kein Fluid in den mindestens zwei Fluidkammern eingebracht ist. Bevorzugt ist die erste Ausdehnung in der Höhenachse des ersten Zustands die minimal mögliche Ausdehnung in der Höhenachse der mindestens zwei Fluidkammern. Daher kann der erste Zustand auch als Grundzustand, nicht-aktiver Zustand oder Aus-Zustand, der mindestens zwei Fluidkammern bzw. der Fluidkammereinheit bezeichnet werden. Die Ausdehnung in der Höhenachse der Fluidkammereinheit bzw. der mindestens zwei Fluidkammern erfolgt nach oben in Richtung des Sitzpolsterteils, da das darüber angeordnete Sitzpolsterteil verformbar/flexibel ausgebildet ist, wohingegen das Sitzplattenteil starr ausgebildet ist. Auf diese Weise ist die Ausdehnung der Fluidkammereinheit bzw. der mindestens zwei Fluidkammern in der Höhenachse durch das Einbringen/Entnehmen des Fluids gesteuert einstellbar bzw. variierbar, wodurch die Mikrobewegungen erzeugt werden, die sich auf das Sitzpolsterteil übertragen und von dort auf eine Person, die auf dem Fahrzeugsitz bzw. dem Sitzpolsterteil sitzt.

Bevorzugt ist also in jede vorgesehene Fluidkammer der Fluidkammereinheit jeweils einzeln und unabhängig von anderen Fluidkammern der Fluidkammereinheit Fluid einbringbar und ist aus jeder vorgesehenen Fluidkammer der Fluidkammereinheit jeweils einzeln und unabhängig von anderen Fluidkammern der Fluidkammereinheit Fluid entnehmbar. Dabei sind die Steuereinheit und die Fluidfördereinheit dazu ausgebildet, das Fluid in jede Fluidkammer der Fluidkammereinheit einzeln und unabhängig mit einer variablen Fluidmenge einzubringen und zu entnehmen, das Fluid mit einer variablen Geschwindigkeit bzw. über einen variablen Zeitraum (variable Fluidförderrate) einzubringen und zu entnehmen und/oder das Fluid zu einem variablen Zeitpunkt einzubringen bzw. zu entnehmen. Somit kann ein sog. Bewegungsmuster über die unterschiedliche Ansteuerbarkeit der mindestens zwei Fluidkammern ermöglicht werden.

Bevorzugt ist die Fluidfördereinrichtung jeweils durch mindestens ein Fluidverbindungselement fluidisch mit jeder der mindestens zwei Fluidkammern individuell verbunden. Bevorzugt handelt es sich bei dem Fluidverbindungselement um einen Schlauch oder dergleichen, welcher geeignet ist das entsprechende Fluid zu transportieren. Durch die Fluidfördereinrichtung, welche mit jeder der mindestens zwei Fluidkammern der Fluidkammereinheit individuell verbunden ist, ist das Fluid steuerbar durch die Steuereinheit jeweils einzeln in die mindestens zwei Fluidkammern einbringbar und/oder entnehmbar. Durch das Vorsehen mehrerer Fluidkammern für die Fluidkammereinheit, welche durch die individuelle fluidische Verbindung mit der Fluidfördereinrichtung separat mit dem Fluid befüllbar und das Fluid entnehmbar ist, können abwechselnde Mikrobewegungen in dem Sitzpolsterteil erzeugt werden und beispielsweise unterschiedliche Bewegungsprogramme definiert werden. Durch die Anordnung mehrerer individueller Fluidkammern, in die durch die Steuereinheit separat Fluid einbringbar und entnehmbar ist, ist die Ausdehnung der Fluidkammereinheit in der Höhenachse bereichsabhängig bzw. abschnittsweise (links/rechts und/oder vorne/hinten) einstellbar bzw. steuerbar. So ist beispielsweise in die mindestens zwei Fluidkammern gleichzeitig das Fluid in beide Fluidkammern einbringbar, gleichzeitig das Fluid aus beiden Fluidkammern entnehmbar, gleichzeitig das Fluid aus einer Fluidkammer entnehmbar und in die andere Fluidkammer einbringbar oder nur aus einer der Fluidkammern das Fluid entnehmbar oder einbringbar.

Gemäß einer bevorzugten Ausführungsform sind die mindestens zwei Fluidkammern bezogen auf eine Breitenachse oder eine Längenachse des Fahrzeugsitzes benachbart angeordnet. Bevorzugt sind die mindestens zwei Fluidkammern der Fluidkammereinheit benachbart in einer Ebene entlang der Längenachse und der Breitenachse bzw. senkrecht zur Höhenachse angeordnet. Somit sind die mindestens zwei Fluidkammern entweder bevorzugt entlang der Längenachse hintereinander (vorne/hinten) oder entlang der Breitenachse nebeneinander (links/rechts) angeordnet. Somit kann die Fläche, in welcher die Ausdehnung in der Höhenachse einstellbar ist, vergrößert werden.

Besonders bevorzugt weist die Fluidkammereinheit vier Fluidkammern (erste, zweite, dritte, vierte Fluidkammer) auf, welche jeweils unabhängig voneinander mit der Fluidfördereinrichtung verbunden sind. Die vier Fluidkammern sind in der Ebene entlang der Längenachse und der Breitenachse (senkrecht zur Höhenachse) angeordnet. Weiter bevorzugt sind die vier Fluidkammern zweiseitig angrenzend nebeneinander angeordnet. Dabei ist bevorzugt eine erste Fluidkammer mit Blick entlang der Längenachse nach vorne rechts von einer zweiten Fluidkammer bzw. sind die erste Fluidkammer und die zweite Fluidkammer entlang der Breitenachse nebeneinander angeordnet, wobei eine dritte Fluidkammer mit Blick entlang der Längenachse nach vorne rechts von einer vierten Fluidkammer angeordnet ist bzw. sind die dritte Fluidkammer und die vierte Fluidkammer entlang der Breitenachse nebeneinander angeordnet. Die erste Fluidkammer und die zweite Fluidkammer sind bezogen auf die Längenachse hinter der dritten Fluidkammer und der vierten Fluidkammer angeordnet, wobei die erste und die dritte sowie die zweite und die vierte Fluidkammer hintereinander angeordnet sind.

Bevorzugt ist eine maximale zweite Ausdehnung der mindestens zwei Fluidkammern in der Höhenachse gleich, wobei auch denkbar wäre, dass diese unterschiedlich wäre. So weisen die mindestens zwei Fluidkammern bevorzugt jeweils eine zweite Ausdehnung in der Höhenachse von bis zu 100 mm, bevorzugt bis zu 75 mm, weiter bevorzugt bis zu 50 mm und insbesondere bevorzugt von 40 mm auf. Die zweite Ausdehnung der Fluidkammern in der Höhenachse kann auch als Kammerhub bezeichnet werden. Bevorzugt ist die Form jeder der Fluidkammern und damit die Form der Fluidkammereinheit beliebig und kann an den vorhandenen Bauraum angepasst werden.

Gemäß einer bevorzugten Ausführungsform ist die Steuereinheit zumindest signaltechnisch mit der Fluidfördereinrichtung verbunden und dazu ausgebildet, die Fluidfördereinrichtung mittels eines pulsweitenmodulierten Steuersignals anzusteuern. Bevorzugt ist die Steuereinheit leistungselektronisch mit der Fluidfördereinrichtung verbunden. Unter einer leistungselektronischen Verbindung wird eine Verbindung verstanden, mittels welcher zu dem Signal bzw. mit dem Signal auch eine Leistung übertragen wird. Somit ist die Steuereinheit dazu ausgebildet, ein pulsweitenmoduliertes Steuersignal an die Fluidfördereinrichtung zu übermitteln. Auf diese Weise ist vorteilhaft die Leistung der Fluidfördereinrichtung und die damit verbundene Fluidmenge, welche in die Fluidkammern eingebracht werden soll, durch die Steuereinheit einstellbar bzw. steuerbar. Bevorzugt ist die Steuereinheit dazu ausgebildet, die Fluidfördereinheit, insbesondere durch das pulsweitenmodulierte Steuersignal, so zu (anzu-)steuern, dass zumindest eine Fluidmenge, eine Einbring-/Entnahme-Fluidrate und/oder eine Fluidförderzeit (bzw. Schrittlänge) für das Fluid, das in die mindestens zwei Fluidkammern der Fluidkammereinheit einbringbar/entnehmbar ist, einstellbar bzw. steuerbar ist. Dies gilt insbesondere bevorzugt individuell für jede der vorgesehenen Fluidkammern der Fluidkammereinheit. Somit ist bevorzugt das Einbringen und das Entnehmen des Fluids in jede der vorgesehenen Fluidkammern der Fluidkammereinheit durch die Steuereinheit separat steuerbar bzw. einstellbar. Besonders bevorzugt ist durch die Steuereinheit eine in die Fluidkammer einzubringende/zu entnehmende Fluidmenge, die Fluidförderrate und der Zeitpunkt des Einbringens/Entnehmens des Fluids für jede Fluidkammer der Fluidkammereinheit unabhängig voneinander steuerbar bzw. einstellbar.

Unter einem pulsweitenmodulierten Steuersignal wird bevorzugt ein Signal verstanden, welches von der Steuereinheit an die damit zumindest signaltechnisch, bevorzugt leistungselektronisch, verbundene Fluidfördereinrichtung übermittelt wird und dazu ausgebildet ist, die Fluidfördereinrichtung anzusteuern, wobei über das pulsweitenmodulierten Steuersignal eine durch die Fluidfördereinrichtung erzeugte Leistung einstellbar bzw. steuerbar ist. Die Pulsweitenmodulation (Pulse-width Modulation = PWM) ist eine Modulationsart bei der eine Spannung, ein Strom oder dergleichen bei einer festen Frequenz zwischen zwei festen Werten wechselt. Die zu übertragene Information ist im Tastverhältnis/Tastgrad (engl. "Duty Cycle") untergebracht. Eine Periode der Pulsweitenmodulation besteht aus dem Puls und der Pause. Der Modulationsgrad wird im Tastverhältnis der Pulslänge zur Periodendauer (Puls + Pause) in Prozent ausgedrückt. Die Erzeugung sowie Anwendung von pulsweitenmodulierten Signalen ist aus dem Stand der Technik hinreichend bekannt, weshalb hier nicht näher darauf eingegangen werden soll.

Gemäß einer bevorzugten Ausführungsform ist die Fluidkammereinheit im Wesentlichen plattenartig ausgebildet. Bevorzugt ist bezogen auf die Höhenachse des Fahrzeugsitzes ein Abdeckelement unterhalb der Fluidkammereinheit und zwischen dem Sitzplattenteil und der Fluidkammereinheit angeordnet. Weiter bevorzugt ist, bezogen auf die Höhenachse des Fahrzeugsitzes, ein Plattenelement oberhalb der Fluidkammereinheit und zwischen dem Sitzpolsterteil und der Fluidkammereinheit angeordnet. Weiter bevorzugt ist, die Fluidkammereinheit bezogen auf die Höhenachse, zwischen dem Abdeckelement und dem Plattenelement angeordnet. Das Abdeckelement und das Plattenelement dienen bevorzugt zum mechanischen Schutz der Fluidkammereinheit bzw. der Fluidkammern vor Beschädigung. Weiterhin ist mittels dem Abdeckelement die Fluidkammereinheit bevorzugt an dem Sitzplattenteil befestigbar. Ebenfalls bevorzugt gewährleistet das Plattenelement, dass die punktuellen Bewegungen (zweiten Ausdehnungen in der Höhenachse) der Fluidkammereinheit bzw. der Fluidkammern möglichst flächig auf das Sitzpolsterteil übertragen werden, d.h. eine flächige Kraftübertragung stattfindet. So führt das Einbringen des Fluids in mindestens eine Fluidkammer der Fluidkammereinheit zur Auslenkung des Plattenelements in der Höhenachse, welche durch Entnahme des Fluids reversibel ist.

Gemäß einer bevorzugten Ausführungsform sind das Abdeckelement und das Plattenelement bezogen auf die Höhenachse des Fahrzeugsitzes zumindest teilweise, einen dazwischenliegenden zweiten Überlappungsbereich ausbildend, überlappend angeordnet. Bevorzugt ist die Fluidkammereinheit zumindest teilweise, bevorzugt vollständig, innerhalb des zweiten Überlappungsbereichs zwischen dem Abdeckelement und dem Plattenelement angeordnet. Weiter bevorzugt sind das Abdeckelement und das Plattenelement zumindest teilweise, bevorzugt vollständig, innerhalb des ersten Überlappungsbereichs zwischen dem Sitzplattenteil und dem Sitzpolsterteil angeordnet. Durch die Ausbildung des zweiten Überlappungsbereichs zwischen dem Abdeckelement und dem Plattenelement in der Höhenachse und der Anordnung der Fluidkammereinheit in dem zweiten Überlappungsbereich kann der mechanische Schutz, die Befestigung und die flächige Kraftübertragung gewährleistet werden.

Gemäß einer bevorzugten Ausführungsform kontaktiert das Abdeckelement das Sitzplattenteil oberseitig. Bevorzugt kontaktiert das Plattenelement das Sitzpolsterteil unterseitig. Besonders bevorzugt ist das Plattenelement frei von Verbindungen gegenüber der Fluidkammereinheit und dem Sitzpolsterteil angeordnet und nur kontaktierend zu der Fluidkammereinheit und dem Sitzpolsterteil angeordnet, sodass die Kraftübertragung durch die Ausdehnung der Fluidkammereinheit bzw. der Fluidkammern in der Höhenachse besonders vorteilhaft stattfinden kann und nicht durch feste Verbindungen beeinträchtigt wird. Weiter bevorzugt weisen das Abdeckelement und das Plattenelement jeweils eine Ausdehnung in der Höhenachse des Fahrzeugsitzes in einem Bereich von 0,1 mm bis 10 mm, bevorzugt in einem Bereich von 0,5 mm bis 8 mm, weiter bevorzugt in einem Bereich von 1 mm bis 6 mm, besonders bevorzugt in einem Bereich von 1,5 mm bis 4 mm und insbesondere bevorzugt von 2 mm auf. Durch diese bevorzugte Ausdehnung in der Höhenachse, auch als Dicke bezeichnet, wird der Platzbedarf in der Höhenachse gering gehalten, da hier kein großer Bauraum zur Verfügung steht. Bevorzugt ist eine Ausdehnung des Abdeckelements und des Plattenelements senkrecht zu der Höhenachse des Fahrzeugsitzes größer als eine Ausdehnung der Fluidkammereinheit senkrecht zu der Höhenachse des Fahrzeugsitzes, um eine Anordnung der Fluidkammereinheit in dem zweiten Überlappungsbereich zu vereinfachen. Bevorzugt sind das Fluidkammerelement, das Abdeckelement und das Plattenelement plattenartig ausgebildet, um einen geringen Platzbedarf in der Höhenachse zu gewährleisten.

Gemäß einer bevorzugten Ausführungsform ist das Abdeckelement mechanisch mit der Fluidkammereinheit verbunden. Weiter bevorzugt besteht das Abdeckelement aus einem flexiblen Material, bevorzugt einem textilen Material, weiter bevorzugt einem Filz. Bevorzugt wird die Fluidkammereinheit durch Verbindungsmittel, wie Nieten, Bolzen, Klammern oder dergleichen mit dem Abdeckelement verbunden. Auf diese Weise kann durch das Abdeckelement eine positionssichere Befestigung der Fluidkammereinheit gegenüber dem Sitzpolsterteil gewährleistet werden. Gemäß einer bevorzugten Ausführungsform ist das Plattenelement planar ausgebildet. Bevorzugt besteht das Plattenelement aus einem flexiblen Material, bevorzugt einem Kunststoff. So ist durch die flexible Ausgestaltung und Biegsamkeit eine Anpassung und optimale Kraftübertragung von der Fluidkammereinheit auf das Sitzpolsterteil ermöglicht. Weiter bevorzugt ist das Plattenelement aus einem geeigneten polymeren Kunststoff ausgebildet, wobei hierzu beispielsweise Polyethylen, Polypropylen, Polyvinylchlorid, Polyurethan, Polyethylenterephthalat, oder dergleichen verwendet werden kann, wobei die Auswahl nicht auf diese Kunststoffe beschränkt sein soll. Bevorzugt ist jedes flexible Material geeignet, welches durch die Bewegungen der Fluidkammereinheit bzw. der Fluidkammern in der Höhenachse verformbar bzw. biegbar ist, um die Bewegungen an das Sitzpolsterteil zu übertragen.

Die Aufgabe der Erfindung wird auch gelöst von einem System, bevorzugt angeordnet in einem Fahrzeug, umfassend einen Fahrzeugsitz nach einem der Ansprüche 1 bis 6 und eine zumindest signaltechnisch mit der Steuereinheit verbundene Aktivierungseinheit, welche ausgebildet ist, ein Aktivierungssignal an die Steuereinheit zu übermitteln, um ein Einbringen und/oder Entnehmen eines Fluids in/aus mindestens eine(r) Fluidkammer von mindestes zwei Fluidkammern einer Fluidkammereinheit zu initiieren.

Unter einer Aktivierungseinheit wird eine Einrichtung verstanden, mittels welcher ein Aktivierungssignal an die Steuereinheit übermittelbar ist, wobei die Steuereinheit in Antwort auf das Aktivierungssignal ein pulsweitenmoduliertes Steuersignal an die Fluidfördereinrichtung ausgibt und die Fluidfördereinrichtung die entsprechende definierte Fluidmenge in die mindestens zwei Fluidkammern der Fluidkammereinheit einbringt und/oder entnimmt.

Gemäß einer bevorzugten Ausführungsform umfasst die Aktivierungseinheit eine manuell betätigbare Bedieneinheit und/oder eine Körperfunktionserkennungseinheit. Bevorzugt ist die Körperfunktionserkennungseinheit dazu ausgebildet, mindestens eine Körperfunktion eines auf dem Fahrzeugsitz sitzenden Person zu erfassen, und weist zumindest einen ersten Sensor auf. Die manuell betätigbare Bedieneinheit kann bevorzugt von einer Person, die auf dem Fahrzeugsitz sitzt, betätigt werden, wodurch das Aktivierungssignal an die Steuereinheit übermittelt wird. Daher kann die Bedieneinheit auch als Ein-/Ausschalteinheit bezeichnet werden. Auf diese Weise kann die Person auf dem Fahrzeugsitz mittels der Bedieneinheit entsprechend der Bedürfnisse festlegen, wann und ob die Fluidkammereinheit aktiv sein soll. Weiter bevorzugt ist durch die Bedieneinheit auch eine Fluidmenge, die in die Fluidkammern eingebracht wird, einstellbar (dies entspricht einer Intensität). Ebenfalls bevorzugt ist mittels der Bedieneinheit eine Fluidförderrate einstellbar, wobei dies einer Fluidmenge pro Zeit entspricht. Dies gilt sowohl für das Einbringen und das Entnehmen des Fluids. Bevorzugt ist die Bedieneinheit so angeordnet, dass sie für die Person, die auf dem Fahrzeugsitz sitzt, einfach erreichbar und bedienbar ist. So ist die Bedieneinheit bevorzugt in/an einem Armaturenbereich, in/an einem Lenkrad, in/an einer Armlehne oder in/an einer Mittelkonsole eines Fahrzeuges, in dem der Fahrzeugsitz angeordnet ist, angeordnet.

Bevorzugt kann die Körperfunktionserkennungseinheit mindestens eine Herzfrequenz, einen Puls, eine Atemfrequenz, eine Blinzelfrequenz der Augenlider, eine Pupillengröße, eine Körpertemperatur oder dergleichen mittels mindestens eines ersten Sensors erfassen. Bevorzugt ist der erste Sensor ein optischer, elektrischer, magnetischer, elektromagnetischer, thermischer, kapazitiver, akustischer oder mechanischer Sensor. Weiter bevorzugt ist der erste Sensor in oder an einer beliebigen Komponente des Fahrzeugsitzes angeordnet, beispielsweise in dem Sitzpolsterteil oder dem Rückenlehnenteil. Bevorzugt kann mittels der erfassten Sensordaten die Körperfunktionserkennungseinrichtung auf eine Körperfunktion schließen, welche wiederrum auf einen (Gesundheits-)Zustand der Person, die auf dem Fahrzeugsitz sitzt, schließen lässt. Bevorzugt veranlasst die Körperfunktionserkennungseinheit automatisch das Übermitteln des Aktivierungssignals an die Steuereinheit, falls ein (Ge-sundheits-)Zustand, wie Müdigkeit, Unkonzentriertheit, Muskelverspannung oder dergleichen erkannt wird, der die Fahrzeugführung beeinträchtigen könnte. Besonders bevorzugt übermittelt die Körperfunktionserkennungseinrichtung automatisch ein Aktivierungssignal an die Steuereinheit, wenn mittels des mindestens einen ersten Sensors Sensordaten erfasst werden, welche auf eine vorbestimmte Körperfunktion schließen lassen. So kann beispielsweise ab einer bestimmten Herzfrequenz, Blinzelhäufigkeit oder einem Puls auf eine bestimmte Körperfunktion, z.B. Müdigkeit geschlossen werden. Bevorzugt ist die Körperfunktionserkennungseinrichtung dazu ausgebildet, mindestens eine Körperfunktion einer auf dem Fahrzeugsitz sitzenden Person basierend auf erfassten Sensordaten mindestens eines ersten Sensors zu ermitteln.

Gemäß einer bevorzugten Ausführungsform umfasst die Steuereinheit eine damit zumindest signaltechnisch verbundene Speichereinheit. Bevorzugt ist auf der Speichereinheit mindestens ein Bewegungsprogramm gespeichert, wobei es sich bei einem Bewegungsprogramm vorteilhaft um eine vorbestimmte Sequenz bzw. einem vorbestimmten Ablauf an Einbringen und Entnehmen von Fluid in mindestens eine Fluidkammer der Fluidkammereinheit handelt. Die Bewegungsprogramme können vorbestimmt sein und unveränderlich auf der Speichereinheit abgespeichert werden oder durch eine Person geändert und/oder erstellt und abgespeichert werden. Bevorzugt sind auf der Speichereinheit die jeweils definierten Fluidmengen zum Einbringen/Entnehmen in die mindestens zwei Fluidkammern gespeichert und durch die Steuereinheit abrufbar. Weiter bevorzugt ist ebenfalls eine Fluidförderrate zum Einbringen/Entnehmen des Fluids für jede der mindestens zwei Fluidkammern auf der Speichereinheit gespeichert. Besonders bevorzugt sind weiter jeweils Zeitpunkte bzw. eine Abfolge für das Einbringen/Entnehmen des Fluids in die mindestens zwei Fluidkammern abgespeichert. Die Aufgabe der Erfindung wird weiter gelöst von einem Verfahren zum Betreiben eines Systems nach Anspruch 7 oder 8, umfassend die Schritte:
a) Empfangen eines Aktivierungssignals ausgehend von einer Aktivierungseinheit durch eine Steuereinheit;
b) Ausgeben eines pulsweitenmodulierten Steuersignals durch die Steuereinheit an eine Fluidfördereinheit;
c) Einbringen eines Fluids in mindestens eine Fluidkammer von mindestes zwei Fluidkammern einer Fluidkammereinheit.

Gemäß einer bevorzugten Ausführungsform umfasst das Verfahren einen weiteren Schritt:
d) Entnehmen des Fluids aus der mindestens einen Fluidkammer der Fluidkammereinheit.

Gemäß einer bevorzugten Ausführungsform können die Schritte c) und d) des Verfahrens beliebig oft wiederholt werden. Auf diese Weise können sich Bewegungsmuster ergeben, die als Bewegungsprogramme auf einer Speichereinheit der Steuereinheit gespeichert werden können. Bevorzugt kann in dem Schritt c) das Fluid in mehrere Fluidkammern gleichzeitig eingebracht werden. Weiter bevorzugt kann in dem Schritt d) das Fluid aus mehreren Fluidkammern gleichzeitig entnommen werden. Bevorzugt können die Schritte c) und d) für unterschiedliche Fluidkammern - es müssen also mindestens zwei Fluidkammern vorgesehen sein - gleichzeitig erfolgen, wobei aus einer Fluidkammer, in die zuvor Fluid eingebracht wurde, Fluid entnommen wird und in eine andere Fluidkammer - in die noch kein Fluid eingebracht wurde oder das Fluid bereits wieder entnommen wurde - Fluid eingebracht wird.

Bevorzugt können in Schritt c) unterschiedliche Fluidmengen in mehrere (mindestens zwei) Fluidkammern eingebracht werden. Ebenfalls bevorzugt können in Schritt d) unterschiedliche Fluidmengen aus mehreren (mindestens zwei) entnommen werden.

Des Weiteren wird die Aufgabe gelöst durch ein Fahrzeug, insbesondere Kraftfahrzeug, umfassend das System nach einem der Ansprüche 11 oder 12.

Im Folgenden wird eine bevorzugte Ausführungsform des erfindungsgemäßen Fahrzeugsitzes, des Systems und des Verfahrens beispielhaft dargestellt, wobei die Erfindung nicht auf dieses Beispiel beschränkt sein soll.

Es ist ein erfindungsgemäßer Fahrzeugsitz vorgesehen, wobei die Fluidkammereinheit vier separate Fluidkammern aufweist, die jeweils individuell mit einer Druckluft-fördernden Pumpe (Fluidfördereinrichtung) fluidisch verbunden sind. Daher können die Fluidkammern in diesem Beispiel auch als Luftkammern bezeichnet werden. Die Fluidkammern sind hiernach im Wesentlichen rechteckig, insbesondere quadratisch, ausgebildet. Die vier Fluidkammern sind dabei in der Ebene der Längenachse und der Breitenachse (senkrecht zur Höhenachse) benachbart angeordnet, wobei die vier Fluidkammern so angeordnet sind, dass sich wiederrum eine rechteckige, insbesondere quadratische Form der Fluidkammereinheit ergibt.

Die Anordnung der vier Fluidkammern der Fluidkammereinheit ist wie folgt schematisch dargestellt (mit Blick entlang der Höhenachse von oben nach unten):

| | |
|---|---|
| Fluidkammer **1** | Fluidkammer **2** |
| Fluidkammer **3** | Fluidkammer **4** |

Bei Erhalt eines Aktivierungssignals von der Aktivierungseinheit durch die Steuereinheit, gibt die Steuereinheit mindestens ein pulsweitenmoduliertes Steuersignal an die Pumpe aus, woraufhin ein vorbestimmtes Bewegungsprogramm ausgeführt wird. Das vorbestimmte Bewegungsprogramm (Tabelle 1), bestehend aus 4 Sequenzen, kann beispielsweise durch eine manuell bedienbare Bedieneinheit ein-/ausgeschaltet und in der Intensität (über die in die Fluidkammern eingebrachte Fluidmenge) über die Pulsweitenmodellierung (PWM) der Pumpe durch das Steuersignal der Steuereinheit dreistufig verändert werden (Tabelle 2 / 3). Die Pumpzeit bzw. Fluidförderzeit (Schrittlänge) beträgt 5 Sekunden bei Verwendung einer Pumpe mit einem charakteristischen Pₘₐₓ von 800 hPa und einer Befüllzeit (gegeben durch die Fluidförderrate) von ≤ 8 s (1l @ 550 hPa).

**Tabelle 1: vorbestimmtes Bewegungsprogramm**

| | **Sequenz** | **Dauer [min]** |
|---|---|---|
| 1 | Links-Rechts Wechsel | 15 |
| 2 | Pause | 5 |
| 3 | Kreuz | 15 |
| 4 | Pause | 25 |
| 5 | Wiederholen der Sequenzen 1-4 | 60 |

Das gemäß der Tabelle 1 dargestellte Bewegungsmuster umfasst fünf Sequenzen. Die Sequenz "Pause" (Sequenzen 2 und 4) bezeichnen dabei den Zustand, wenn alle vier Fluidkammern eine minimale Ausdehnung in der Höhenachse (sog. Grundzustand) aufweisen (im Wesentlichen kein Fluid eingebracht ist bzw. alles Fluid entnommen ist), wenn im Wesentlichen das gesamte Fluid aus den Fluidkammern entnommen wurde.

Die Sequenz 1 "Rechts-Links Wechsel" bezeichnet eine Abfolge von Einbring- und Entnahme-Schritten des Fluids (analog zu den Schritten c) und d) des erfindungsgemäßen Verfahrens) gemäß:
Schritt 1: Einbringen des Fluids in die Fluidkammer 1 und in die Fluidkammer 3;
Schritt 2: Entnehmen des Fluids aus der Fluidkammer 1 und aus der Fluidkammer 3 und Einbringen des Fluids in die Fluidkammer 2 und in die Fluidkammer 4;
Schritt 3: Einbringen des Fluids in die Fluidkammer 1 und in die Fluidkammer 3 und Entnehmen des Fluids aus der Fluidkammer 2 und aus der Fluidkammer 4.

Die Sequenz 3 "Kreuz" bezeichnet eine Abfolge von Einbring- und Entnahme-Schritten des Fluids (analog zu den Schritten c) und d) des erfindungsgemäßen Verfahrens) gemäß:
Schritt 1: Einbringen des Fluids in die Fluidkammer 1;
Schritt 2: Entnehmen des Fluids aus der Fluidkammer 1 und Einbringen des Fluids in die Fluidkammer 4;
Schritt 3: Entnehmen des Fluids aus der Fluidkammer 1 und aus der Fluidkammer 4 und Einbringen des Fluids in die Fluidkammer 2;
Schritt 4: Entnehmen des Fluids aus der Fluidkammer 1, aus der Fluidkammer 4 und aus der Fluidkammer 2 und Einbringen des Fluids in die Fluidkammer 3;
Schritt 5: Entnehmen des Fluids aus der Fluidkammer 4, aus der Fluidkammer 2 und aus der Fluidkammer 3 und Einbringen des Fluids in die Fluidkammer 1.

Nach den Sequenzen 1 und 3 wird aus jeder Fluidkammer das Fluid entnommen, so dass sich jede Fluidkammer im ersten Zustand befindet. Die Schritte 3 und 4 der Sequenz 1 sowie die Schritte 2 bis 5 der Sequenz 3 können bevorzugt beliebig wiederholt werden. Durch die Sequenz 1 wird die Fluidkammereinheit abwechselnd "links und rechts" aufgepumpt bzw. entleert und durch die Sequenz 3 wird immer eine Fluidkammer der Fluidkammereinheit aufgepumpt, wobei in den Schritten 2 und 4 jeweils in die diagonal angeordnete ("über Kreuz") Fluidkammer das Fluid eingebracht wird.

Wie oben bereits erwähnt, ist die Intensität mittels der Fluidmenge, die in die jeweilige Fluidkammer eingebracht wird, sowie die Pumpzeit (Fluidförderrate bzw. Schrittlänge) einstellbar. Die Intensität bzw. Fluidmenge wird bevorzugt durch das pulsweitenmodulierte Steuersignal von der Steuereinheit gesteuert bzw. eingestellt. Dabei lässt sich die Intensität der Sequenz 1 (Tabelle 2) und der Sequenz 3 (Tabelle 3) in drei Stufen folgendermaßen einstellen:

**Tabelle 2: Sequenzparameter der Sequenz 1**

| | | | |
|---|---|---|---|
| **Intensität** | 1 | 2 | 3 |
| **Schrittlänge [s]** | 5 | 5 | 5 |
| **PWM-Steuersignal [%]** | 70 | 85 | 100 |

**Tabelle 3: Sequenzparameter der Sequenz 3**

| | | | |
|---|---|---|---|
| **Intensität** | 1 | 2 | 3 |
| **Schrittlänge [s]** | 5 | 5 | 5 |
| **PWM-Steuersignal [%]** | 60 | 85 | 100 |

Das aufgeführte Beispiel zeigt die Möglichkeit, welche die Fluidkammereinheit bietet, indem mehrere Fluidkammern unabhängig voneinander mit Fluid gefüllt und geleert werden, wodurch sich Bewegungsmuster erzeugen lassen, die die beteiligten Muskeln unterschiedlich ansprechen und durch die Abwechslung, die Aufmerksamkeit hoch halten.

Die Ausführungen bezüglich des erfindungsgemäßen Fahrzeugsitzes sollen mutatis mutandis für das erfindungsgemäße System, das Verfahren und das Fahrzeug und umgekehrt gelten.

Weitere Vorteile, Ziele und Eigenschaften vorliegender Erfindung werden anhand anliegender Zeichnung und nachfolgender Beschreibung erläutert, in welchen eine Steuervorrichtung beispielhaft dargestellt und beschrieben ist.

In der Zeichnung zeigen:
- Fig. 1: einen Fahrzeugsitz gemäß einer bevorzugten Ausführungsform der Erfindung;
- Fig. 2a, 2b: verschiedene Explosionsdarstellungen des Fahrzeugsitzes gemäß Fig. 1;
- Fig. 3: eine Detailansicht eines Sitzplattenteils mit einer Fluidkammereinheit gemäß Fig. 1;
- Fig. 4a, 4b: eine Detailansicht einer Fluidkammereinheit mit und ohne Plattenele-ment gemäß einer bevorzugten Ausführungsform in einem ersten Zu-stand und in einem zweiten Zustand;
- Fig. 5a, 5b: eine Querschnittsansicht eines Sitzoberteils gemäß einer bevorzugten Ausführungsform in einem ersten und einem zweiten Zustand;
- Fig. 6: eine Draufsicht auf eine Fluidkammereinheit in einem ausgebauten Zustand gemäß einer bevorzugten Ausführungsform;
- Fig. 7: eine schematische Darstellung eines Systems gemäß einer bevorzugten Ausführungsform.

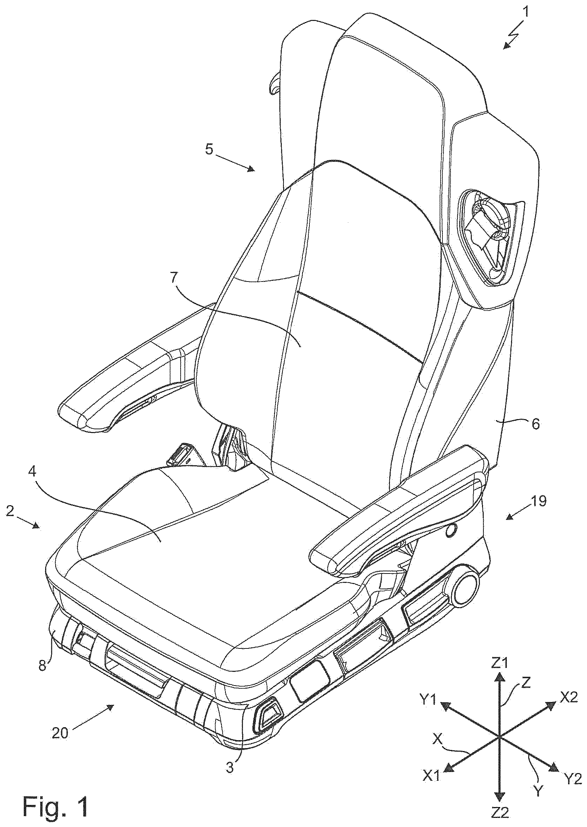

Die Fig. 1 zeigt einen Fahrzeugsitz 1 gemäß einer bevorzugten Ausführungsform der Erfindung. Der Fahrzeugsitz 1 ist bevorzugt in einem Kraftfahrzeug (nicht dargestellt), besonders bevorzugt in einem Nutzfahrzeug angeordnet. In den Figuren 1 bis 5 ist jeweils keine Steuereinheit und keine Fluidfördereinrichtung dargestellt. Die Anordnung dieser Komponenten im oder am Fahrzeugsitz 1 bzw. im Fahrzeug.

Der Fahrzeugsitz 1 erstreckt sich entlang einer Höhenachse Z, einer Breitenachse Y und einer Längenachse X, wobei den jeweiligen Achsen jeweils zwei Richtungen zuzuordnen sind. So umfasst die Höhenachse Z die Richtungen nach oben Z1 und nach unten Z2. Die Breitenachse Y umfasst die Richtungen nach rechts Y1 und nach links Y2. Die Längenachse X umfasst die Richtungen nach vorne X1 und nach hinten X2.

Der Fahrzeugsitz 1 gemäß Fig. 1 weist ein Sitzoberteil 2, umfassend ein Sitzplattenteil 3 und ein Sitzpolsterteil 4, und ein Rückenlehnenteil 5, umfassend ein Rückenplattenteil 6 und ein Rückenpolsterteil 7, auf. Das Sitzoberteil 2 und das Rückenlehnenteil 5 sind zumindest mechanisch miteinander verbunden, wobei das Sitzoberteil 2 und das Rückenlehnenteil 5 bevorzugt relativ zueinander bewegbar sind. Das Sitzoberteil 5 ist zumindest mechanisch mit einem Sitzbasisteil 8 angeordnet, wobei das Sitzoberteil 2 bezogen auf die Höhenachse Z oberhalb des Sitzbasisteils 8 angeordnet ist. Das Sitzbasisteil 8 kann entweder direkt an einer Fahrzeugkarosserie (nicht dargestellt) angeordnet sein oder über eine Dämpfungs-und/oder Federungseinrichtung (nicht dargestellt) an dieser befestigt sein. Das Sitzpolsterteil 4 ist bezogen auf die Höhenachse Z des Fahrzeugsitzes 1 oberhalb der Sitzplattenteils 3 angeordnet, wobei das Sitzpolsterteil 4 an dem Sitzplattenteil 3 befestigt ist.

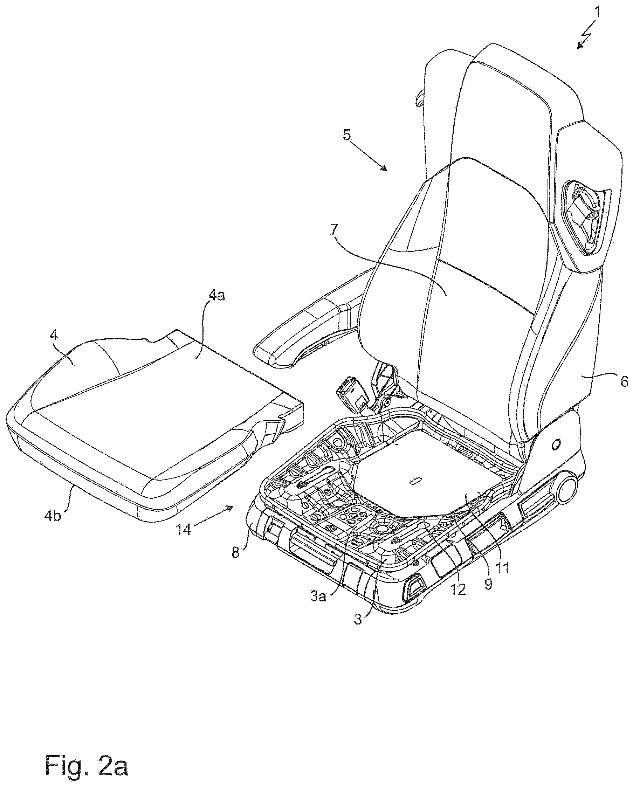

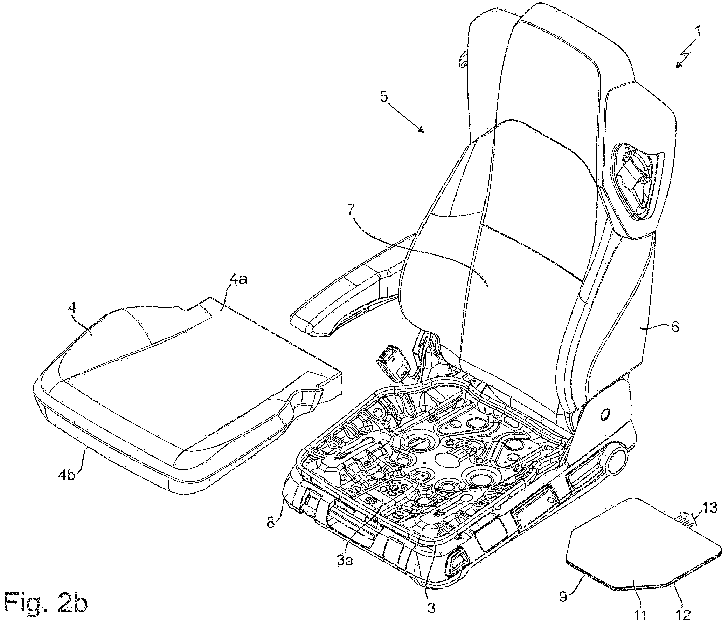

In der Fig. 2 ist der Fahrzeugsitz 1 gemäß Fig. 1 in einer Explosionsdarstellung, das Sitzpolsterteil 4 betreffend, gezeigt. Das Sitzpolsterteil 4 weist eine Oberseite 4a und eine Unterseite 4b auf. In einem zusammengebauten Zustand ist das Sitzpolsterteil 4 über die Unterseite 4b an einer Oberseite 3a des Sitzplattenteils 3 befestigt. Gemäß der gezeigten Ausführungsform sind das Sitzplattenteil 3 und das Sitzpolsterteil 4 bezogen auf die Höhenachse Z im Wesentlichen deckungsgleich angeordnet und bilden einen dazwischenliegenden ersten Überlappungsbereich 14 aus. Der erste Überlappungsbereich 14 ist bezogen auf die Höhenachse Z zwischen dem Sitzplattenteil 3 und dem Sitzpolsterteil 4 angeordnet und ist entlang der Höhenachse Z nach oben Z1 durch die Unterseite 4b des Sitzpolsterteils 4 und nach oben Z2 durch die Oberseite 3a des Sitzplattenteils 3 begrenzt.

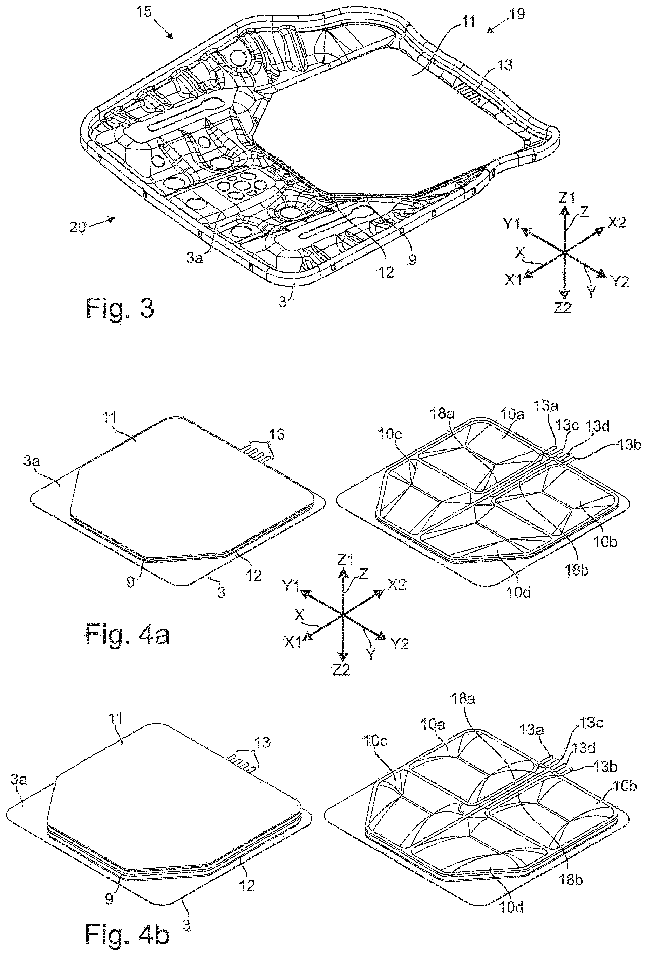

Weiterhin ist eine Fluidkammereinheit 9 zusammen mit einem Plattenelement 11 und einem Abdeckelement 12 in dem ersten Überlappungsbereich 14 zwischen dem Sitzpolsterteil 4 und dem Sitzplattenteil 3 angeordnet. Die Fluidkammereinheit 9, das Plattenelement 11 und das Abdeckelement 12 sind vollständig innerhalb des ersten Überlappungsbereichs 14 angeordnet. Das Plattenelement 11 ist dabei die Unterseite 4b des Sitzpolsterteils und das Abdeckelement 12 die Oberseite 3a des Sitzplattenteils 3 kontaktierend angeordnet. Die Fluidkammereinheit 9 ist bezogen auf die Höhenachse Z zwischen dem Plattenelement 11 und dem Abdeckelement 12 angeordnet. Eine genauere Beschreibung der Fluidkammereinheit 9, des Plattenelements 11 und des Abdeckelements 12 und deren Anordnung folgt in den Figuren 4 bis 6.

Die Fluidkammereinheit 9, das Plattenelement 11 und das Abdeckelement 12 sind bezogen auf eine Längenachse X in einem hinteren Abschnitt 19 des Sitzoberteils 2 bzw. des Sitzplattenteils 3 und der Sitzpolsterteils 4 angeordnet, wobei der hintere Abschnitt 19 bezogen auf die Längenachse X vor einem vorderen Abschnitt 20 des Sitzoberteils 2 bzw. des Sitzplattenteils 3 und des Sitzpolsterteils 4 angeordnet ist. Diese Anordnung ist bevorzugt, da sich ein Gesäß- und/oder unterer Rückenbereich einer Person, welche auf dem Fahrzeugsitz 1 bzw. dem Sitzoberteil 2 bzw. dem Sitzpolsterteil 4 sitzt, im Wesentlichen in dem hinteren Abschnitt 19 befindet, wobei die Oberschenkel der Person im Wesentlichen in dem vorderen Abschnitt 20 angeordnet sind. Somit sind eine Bandscheibenelastizität und Muskelverspannungen im Gesäßbereich besonders effektiv förderbar bzw. reduzierbar.

Die Fig. 3 zeigt eine weitere Explosionsdarstellung, das Sitzpolsterteil 4, die Fluidkammereinheit 9, das Plattenelement 11 und das Abdeckelement 12 betreffend, des Fahrzeugsitzes 1 gemäß Fig. 1. In Fig. 3 sind weiterhin vier Fluidverbindungselement 13 zu sehen, welche mit der Fluidkammereinheit 9 bzw. Fluidkammern der Fluidkammereinheit 9 zumindest fluidisch verbunden sind. Eine genauere Erläuterung folgt in den Figuren 4 bis 6.

In der Fig. 4 ist eine Detailansicht des Sitzplattenteils 3 mit der Fluidkammereinheit 9, dem Plattenelement 11 und dem Abdeckelement 12 gemäß der in Fig. 1 gezeigten Ausführungsform in einem eingebauten Zustand dargestellt.

Gemäß der gezeigten Ausführungsform ist das Sitzplattenteil 3 als Sitzschalenteil ausgebildet, wobei das Sitzschalenteil auf der Oberseite 3a einen nach oben Z1 hin offenen Bereich 15 einschließt. So ist das Sitzpolsterteil 4 zumindest teilweise innerhalb dieses eingeschlossenen Bereichs 15 angeordnet. Die Fluidkammereinheit 9, das Plattenelement 11 und das Abdeckelement 12 sind vollständig in diesem eingeschlossenen Bereich 15 angeordnet. Weiterhin ist der erste Überlappungsbereich 14 ebenfalls vollständig in diesem eingeschlossenen Bereich 15, ausgebildet durch das als Sitzschalenteil ausgebildete Sitzplattenteil 3, angeordnet. Ferner ist zu sehen, dass die Fluidkammereinheit 9, das Plattenelement 11 und das Abdeckelement 12 jeweils plattenartig ausgebildet sind und somit in der Höhenachse einen minimalen Bauraum benötigen. Die Fluidkammereinheit 9, das Plattenelement 11 und das Abdeckelement 12 erstrecken sich im Wesentlichen in einer Ebene entlang der Längenachse X und der Breitenachse Y.

Die Figuren 4a und 4b zeigen jeweils ein Sitzplattenteil 3, auf dessen Oberseite 3a ein Abdeckelement 12 angeordnet ist, wobei in der Höhenachse Z darüber eine Fluidkammereinheit 9 angeordnet ist. In den Figuren 4a und 4b ist jeweils auf der linken Seite über der Fluidkammereinheit 9 in der Höhenachse Z ein Plattenelement 11 angeordnet, wobei auf der rechten Seite das Plattenelement 11 nicht dargestellt ist und ein freier Blick von oben auf die Fluidkammereinheit 9 möglich ist. Ferner sind in den Figuren 4a und 4b (jeweils links) das Abdeckelement 12 und das Plattenelement 11 im Wesentlichen deckungsgleich angeordnet, wobei das Abdeckelement 12 und das Plattenelement 11 bezogen auf die Höhenachse Z des Fahrzeugsitzes 1, einen dazwischenliegenden zweiten Überlappungsbereich 16 ausbildend, überlappend angeordnet sind. Die Fluidkammereinheit 9 ist vollständig innerhalb des zweiten Überlappungsbereichs 16 zwischen dem Abdeckelement 12 und dem Plattenelement 11 angeordnet.

Die Figuren 4a und 4b zeigen rechtsseitig jeweils, dass die Fluidkammereinheit 4 eine erste Fluidkammer 10a, eine zweite Fluidkammer 10b, eine dritte Fluidkammer 10c und eine vierte Fluidkammer 10d aufweist. Dabei sind die erste 10a und die zweite Fluidkammer 10b im Wesentlichen quadratisch ausgebildet, wobei die dritte 10c und die vierte Fluidkammer 10d im Wesentlichen fünfeckig ausgebildet sind. Bevorzugt ist die jeweilige Form der Fluidkammern 10a-d beliebig ausgestaltbar und kann den gegebenen Bauraum des Fahrzeugsitzes 1 zwischen dem Sitzpolsterteil 4 und dem Sitzplattenteil 3 angepasst werden.

Die erste Fluidkammer 10a und die zweite Fluidkammer 10b sind entlang der Breitenachse Y benachbart angeordnet. Die dritte Fluidkammer 10c und die vierte Fluidkammer 10d sind ebenfalls entlang der Breitenachse benachbart angeordnet. Ferner sind die erste Fluidkammer 10a und die dritte Fluidkammer 10c entlang der Längenachse X benachbart angeordnet. Die zweite Fluidkammer 10b und die vierte Fluidkammer 10d sind dabei entlang der Längenachse X benachbart angeordnet. Dabei sind die dritte Fluidkammer 10c und die vierte Fluidkammer 10d in der Längenachse gesehen vor X1 der ersten Fluidkammer 10a und der zweiten Fluidkammer 10b angeordnet.

Jede der Fluidkammern 10a-d ist individuell fluidisch mit einer Fluidfördereinrichtung (hier nicht dargestellt) verbunden, wobei die Verbindung jeweils über ein Fluidverbindungselement 13 erfolgt. Die erste Fluidkammer 10a ist über ein erstes Fluidverbindungselement 13a, die zweite Fluidkammer 10b ist über ein zweites Fluidverbindungselement 13b, die dritte Fluidkammer 10c ist über ein drittes Fluidverbindungselement 13c und die vierte Fluidkammer 10d ist über ein viertes Fluidverbindungselement 13d mit der Fluidfördereinrichtung fluidisch verbunden. Auf diese Weise ist ein Fluid in jede der vier Fluidkammern 10a-d individuell einbringbar und aus jeder der vier Fluidkammern 10a-d individuell entnehmbar.

Die fluidische Verbindung der Fluidkammern 10a-10d durch die Fluidverbindungselemente erfolgt bezogen auf die Längenasche X an einem hinteren Ende 17 der Fluidkammereinheit 9, an dem die erste Fluidkammer 10a und die zweite Fluidkammer 10b angeordnet sind, wobei das hintere Ende 17 in der Längenachse X nach hinten X2 gerichtet ist. So weist die Fluidkammereinheit 9 einen ersten Fluidkanal 18a und einen zweiten Fluidkanal 18b auf, wobei der erste Fluidkanal 18a die dritte Fluidkammer 10c mit dem dritten Fluidverbindungselement 13c und der zweite Fluidkanal 18b die vierte Fluidkammer 10d mit dem vierten Fluidverbindungselement 10d fluidisch verbindet. Der erste 18a und der zweite Fluidkanal 18b verlaufen parallel zueinander zwischen der ersten 10a und der zweiten Fluidkammer 10b entlang der Längenachse X von dem hinteren Ende 17 nach vorne (Richtung X1).

In der Fig. 4a ist die Fluidkammereinheit 9 bzw. jede Fluidkammer 10a-d in einem ersten Zustand dargestellt, wobei die Fluidkammereinheit 9 bzw. die Fluidkammern 10a-d in dem ersten Zustand eine erste Ausdehnung in der Höhenachse Z des Fahrzeugsitzes 1 aufweisen. Diese erste Ausdehnung stellt eine minimale Ausdehnung in der Höhenachse dar und entspricht einem im Wesentlichen vollständig von Fluid entleerten Zustand. In der Fig. 4b ist die Fluidkammereinheit 9 bzw. jede Fluidkammer 10a-d in einem zweiten Zustand dargestellt, wobei die Fluidkammereinheit 9 bzw. die Fluidkammern 10a-d in dem zweiten Zustand eine zweite Ausdehnung in der Höhenachse Z des Fahrzeugsitzes 1 aufweisen. In diesem zweiten Zustand ist eine definierte Fluidmenge in jede Fluidkammer 10a-d eingebracht. Die zweite Ausdehnung in der Höhenachse Z in dem zweiten Zustand gemäß Fig. 4b ist größer als die erste Ausdehnung in der Höhenachse Z in dem ersten Zustand gemäß Fig. 4a.

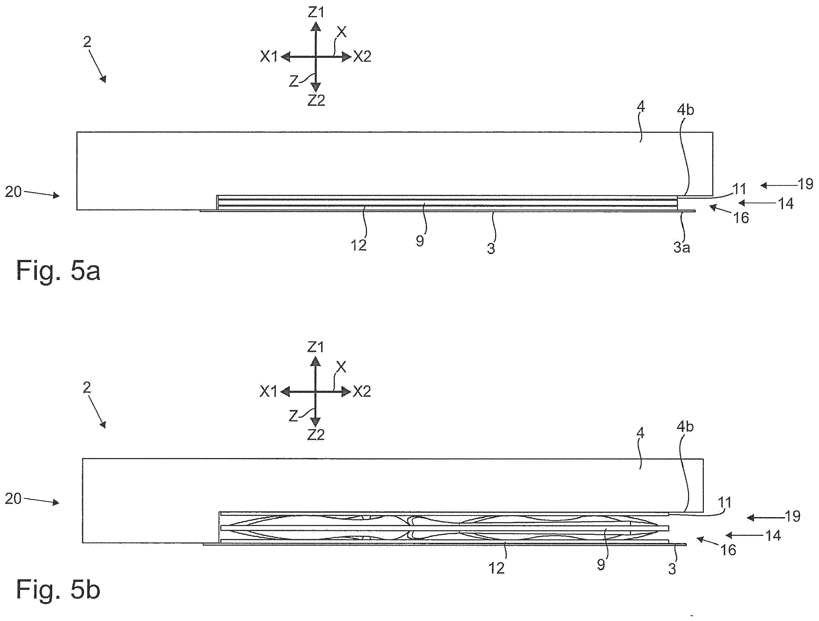

Die Figuren 5a und 5b zeigen jeweils eine Querschnittsansicht entlang der Längsachse X mittig durch einen Fahrzeugsitz 1 bezogen auf die Breitenachse Y eines Sitzoberteils 2 mit einem Sitzplattenteil 3 und einem in der Höhenachse Z oberhalb angeordneten Sitzpolsterteil 4. Das Sitzoberteil 2, sowie das Sitzpolsterteil 4 und das Sitzplattenteil 3 erstrecken sich hauptsächlich in einer Ebene entlang der Längenachse X und der Breitenachse Y, wobei eine Ausdehnung in der Höhenachse Z geringer ist.

Innerhalb eines Überlappungsbereichs 14, der sich bezogen auf die Höhenachse Z zwischen dem Sitzplattenteil 3 und dem Sitzpolsterteil 4 befindet und durch die zumindest teilweise überlappendende Anordnung des Sitzplattenteils 3 und des Sitzpolsterteils 4 bezogen auf die Höhenachse Z ausgebildet ist, ist ein Abdeckelement 12, eine Fluidkammereinheit 9 und ein Plattenelement 11 angeordnet. Das Abdeckelement 12, die Fluidkammereinheit 9 und das Plattenelement 11 sind vollständig innerhalb des ersten Überlappungsbereichs 14 zwischen dem Sitzplattenteil 3 und dem Sitzpolsterteil 4 angeordnet.

Der erste Überlappungsbereich wird bezogen auf die Höhenachse Z nach oben Z1 durch die Unterseite 4a des Sitzpolsterteils 4 und nach unten Z2 durch die Oberseite 3a des Sitzplattenteils 3a begrenzt. In der Längenachse X wird der erste Überlappungsbereich 14 nach vorne X1 durch das Sitzpolsterteil 4 begrenzt. Das Sitzpolsterteil 4 erstreckt sich dabei in dem vorderen Abschnitt 20 des Sitzoberteils 2 bezogen auf die Höhenachse Z weiter nach unten Z2 als in dem hinteren Abschnitt 19, wobei das Sitzpolsterteil 4 in dem vorderen Abschnitt nach unten Z2 bündig (kontaktierend) mit dem Sitzplattenteil 3 abschließt. In dem hinteren Abschnitt 19 ist der erste Überlappungsbereich 16 offen ausgebildet, d.h. das Sitzpolsterteil 4 und das Sitzplattenteil 3 sind nicht kontaktierend ausgebildet, wodurch die Fluidkammereinheit 9 vorteilhaft leicht zugänglich ist, um beispielsweise eine Fluidfördereinrichtung mittels Fluidverbindungselementen 13 fluidisch mit der Fluidkammereinheit verbinden zu können.

Das Abdeckelement 12 ist in der Höhenachse Z über dem Sitzplattenteil 3, dessen Oberseite 3a kontaktierend, angeordnet und an diesem befestigt. Weiterhin ist das Abdeckelement 12 kraftschlüssig, formschlüssig oder stoffschlüssig mit dem Sitzplattenteil 3 verbunden. Das Plattenelement 11 ist hingegen bezogen auf die Höhenachse Z unterhalb des Sitzpolsterteils 4, an dessen Unterseite 4b kontaktierend angeordnet, wobei das Plattenelement 11 und das Sitzpolsterteil 4 frei von einer Verbindung sind. Das Abdeckelement 12 und das Plattenelement 11 sind bezogen auf die Höhenachse Z vollständig, einen dazwischenliegenden zweiten Überlappungsbereich 16 ausbildend, überlappend angeordnet, wobei die Fluidkammereinheit 9 vollständig innerhalb des zweiten Überlappungsbereichs 16 zwischen dem Abdeckelement 12 und dem Plattenelement 11 angeordnet ist. Der zweite Überlappungsbereich 16 ist vollständig innerhalb des ersten Überlappungsbereichs 14 angeordnet. Die Fluidkammereinheit 9 ist an dem Abdeckelement 12 befestigt, wobei das Plattenelement 11 und die Fluidkammereinheit 9 frei von Verbindungen sind.

In der Fig. 5a ist die Fluidkammereinheit 9 in einem ersten Zustand dargestellt. In dem ersten Zustand weist die Fluidkammereinheit 9 eine erste Ausdehnung in der Höhenachse Z auf. Die erste Ausdehnung in der Höhenachse Z der Fluidkammereinheit 9 entspricht dabei einer minimalen Ausdehnung der Fluidkammereinheit 9 in der Höhenachse Z. Die Fluidkammereinheit 9 befindet sich in dem ersten Zustand, wenn im Wesentlichen ein Fluid vollständig entnommen ist.

Die Fig. 5b zeigt hingegen die Fluidkammereinheit 9 in einem zweiten Zustand und eine zweite Ausdehnung in der Höhenachse Z aufweisend. Die zweite Ausdehnung der Fluidkammereinheit 9 in der Höhenachse Z tritt auf, wenn Fluid in die Fluidkammereinheit 9 eingebracht ist. Die zweite Ausdehnung der Fluidkammereinheit 9 ist dabei größer als die erste Ausdehnung der Fluidkammereinheit 9 in der Höhenachse Z.

Die Ausdehnung der Fluidkammereinheit 9 (bzw. der Fluidkammern) erfolgt ausgehend von dem Sitzplattenteil 3 in der Höhenachse Z nach oben Z1 in Richtung des Sitzpolsterteils 4. Durch die Ausdehnung der Fluidkammereinheit 9 in der Höhenachse Z von dem ersten Zustand in den zweiten Zustand kommt es zu einer reversiblen Verformung des Sitzpolsterteils 4, wobei die Bewegung/Auslenkung der Fluidkammereinheit 9 in der Höhenachse Z durch das flexible Plattenelement 11 flächig auf das Sitzpolsterteil 4 übertragen wird. Von dem Sitzpolsterteil 4 wird die Bewegung/Auslenkung wiederrum auf eine Person, die auf dem Sitzpolsterteil 4 bzw. dem Fahrzeugsitz 1 sitzt, übertragen.

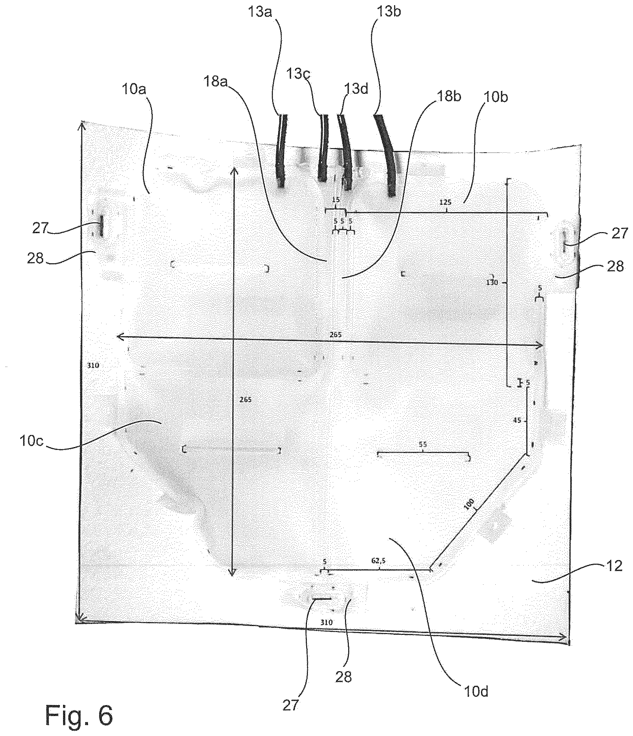

Die Fig. 6 zeigt eine Fluidkammereinheit 9 mit vier Fluidkammern 10a-d. Die Fluidkammereinheit 9 ist mittels Befestigungselementen 27 (in dieser Ausführungsform drei Befestigungselementen) an dem in der Höhenachse Z darunterliegenden Abdeckelement 12 befestigt. Die Befestigungselemente 27 sind bevorzugt als Klammern ausgebildet. Die Befestigungselemente 27 befestigten die Fluidkammereinheit 9 über Befestigungsabschnitte 28 der Fluidkammereinheit 9, die umfänglich an der Fluidkammereinheit 9 angeordnet sind, mit dem Abdeckelement 12.

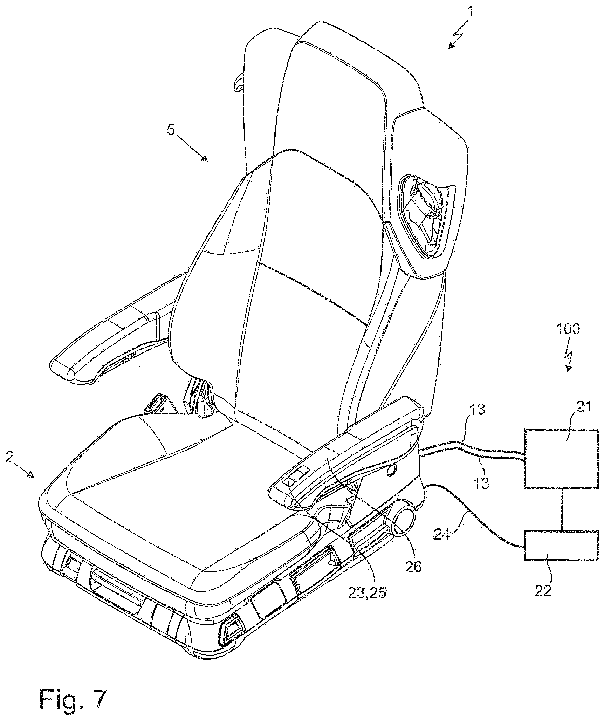

In Fig. 7 ist eine bevorzugte Ausführungsform des erfindungsgemäßen Systems 100 schematisch dargestellt. Das System 100 umfasst den erfindungsgemäßen Fahrzeugsitz 1 mit einer Fluidfördereinrichtung 21 und einer Steuereinheit 22, und eine Aktivierungseinheit 25 mit einer Bedieneinheit 23.

Die Steuereinheit 22 ist zumindest signaltechnisch über eine signaltechnische Verbindung 24 mit der Aktivierungseinheit 25 bzw. der Bedieneinheit 23 verbunden und zumindest signaltechnisch, bevorzugt leistungselektronisch, mit der Fluidfördereinrichtung 21. Die Fluidfördereinrichtung 21 ist über mindestens zwei Fluidverbindungselemente 13 mit der Fluidkammereinheit 9 bzw. jeweils unabhängig voneinander über jeweils ein Fluidverbindungselement 13 mit einer der mindestens zwei Fluidkammern 10 verbunden.

Die Aktivierungseinheit 25 und die Bedieneinheit 23 sind gemäß der gezeigten Ausführungsform zusammen in einer Armlehne 26 des Fahrzeugsitzes 1 angeordnet. In dieser Position kann die Bedieneinheit besonders einfach durch eine auf dem Fahrzeugsitz 1 sitzende Person manuell betätigt werden. Die Aktivierungseinheit 25 ist dazu ausgebildet, bei Betätigung der Bedieneinheit 23 ein Aktivierungssignal über die signaltechnische Verbindung 24 an die Steuereinheit 22 zu übermitteln, woraufhin die Steuereinheit ein pulsweitenmoduliertes Steuersignal an die Fluidfördereinrichtung 21 ausgibt und ein Einbringen/Entnehmen eines Fluids in mindestens ein Fluidkammer 10 von mindestens zwei Fluidkammern 10 einer Fluidkammereinheit 9 stattfindet.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Fahrzeugsitz
- 100: System
- 2: Sitzoberteil
- 3: Sitzplattenteil
- 3a: Oberseite des Sitzplattenelements
- 3b: Unterseite des Sitzplattenelements
- 4: Sitzpolsterteil
- 4a: Oberseite des Sitzpolsterteils
- 4b: Unterseite des Sitzpolsterteils
- 5: Rückenlehnenteil
- 6: Rückenplattenteil
- 7: Rückenpolsterteil
- 8: Sitzbasisteil
- 9: Fluidkammereinheit
- 10: Fluidkammer
- 10a, b, c, d: Fluidkammern
- 11: Plattenelement
- 12: Abdeckelement
- 13: Fluidverbindungselement
- 13a, b, c, d: Fluidverbindungselemente
- 14: erster Überlappungsbereich
- 15: Bereich
- 16: zweiter Überlappungsbereich
- 17: hinteres Ende der Fluidkammereinheit
- 18a, b: Fluidkanal
- 19: hinterer Abschnitt
- 20: vorderer Abschnitt
- 21: Fluidfördereinrichtung
- 22: Steuereinheit
- 23: Bedieneinheit
- 24: signaltechnische Verbindung
- 25: Aktivierungseinheit
- 26: Armlehne
- 27: Befestigungselemente
- 28: Befestigungsabschnitte

## Patentansprüche

1. Fahrzeugsitz (1) mit einem Sitzoberteil (2) zum Aufnehmen einer Person, welches ein Sitzplattenteil (3) und ein Sitzpolsterteil (4) umfasst, wobei das Sitzpolsterteil (4) bezogen auf eine Höhenachse (Z) des Fahrzeugsitzes (1) oberhalb des Sitzplattenteils (3) angeordnet ist und wobei das Sitzplattenteil (3) und das Sitzpolsterteil (4) bezogen auf die Höhenachse (Z) zumindest teilweise, einen dazwischenliegenden ersten Überlappungsbereich (14) ausbildend, überlappend angeordnet sind,
**dadurch gekennzeichnet, dass**
eine Fluidkammereinheit (9) mit mindestens zwei Fluidkammern (10) zumindest teilweise innerhalb des ersten Überlappungsbereichs (14) zwischen dem Sitzplattenteil (3) und dem Sitzpolsterteil (4) angeordnet ist, wobei eine Steuereinheit (22) vorgesehen ist, dazu ausgebildet, ein Fluid in die mindestens zwei Fluidkammern (10) einzubringen und zu entnehmen, sodass eine Ausdehnung in der Höhenachse (Z) der mindestens zwei Fluidkammern (10) steuerbar ist.

2. Fahrzeugsitz (1) nach Anspruch 1,
**dadurch gekennzeichnet, dass**
die mindestens zwei Fluidkammern (10) jeweils dazu ausgebildet sind, das Fluid aufzunehmen, und wobei eine zumindest signaltechnisch mit der Steuereinheit (22) verbundene Fluidfördereinrichtung (21) vorgesehen ist, die zumindest fluidisch mit jeder der mindestens zwei Fluidkammern (10) unabhängig verbunden ist und dazu ausgebildet ist, das Fluid in und/oder aus jeder der mindestens zwei Fluidkammern (10) zu transportieren, wobei das Fluid in jede der mindestens zwei Fluidkammern (10) individuell einbringbar und aus jeder der mindestens zwei Fluidkammern (10) individuell entnehmbar ist.

3. Fahrzeugsitz (1) nach Anspruch 1 oder 2,
**dadurch gekennzeichnet, dass**
die mindestens zwei Fluidkammern (10) bezogen auf eine Breitenachse (Y) oder eine Längenachse (X) des Fahrzeugsitzes benachbart angeordnet sind.

4. Fahrzeugsitz (1) nach einem der vorhergehenden Ansprüche,
**dadurch gekennzeichnet, dass**
die Fluidkammereinheit (9) im Wesentlichen plattenartig ausgebildet ist, wobei bezogen auf die Höhenachse (Z) ein Abdeckelement (12) unterhalb der Fluidkammereinheit (9) und zwischen dem Sitzplattenteil (3) und der Fluidkammereinheit (9) angeordnet ist und wobei bezogen auf die Höhenachse (Z) ein Plattenelement (11) oberhalb der Fluidkammereinheit (9) und zwischen dem Sitzpolsterteil (4) und der Fluidkammereinheit (9) angeordnet ist, wobei die Fluidkammereinheit (9) bezogen auf die Höhenachse (Z) zwischen dem Abdeckelement (12) und dem Plattenelement (11) angeordnet ist.

5. Fahrzeugsitz (1) nach Anspruch 4,
**dadurch gekennzeichnet, dass**
das Abdeckelement (12) und das Plattenelement (11) bezogen auf die Höhenachse (Z) zumindest teilweise, einen dazwischenliegenden zweiten Überlappungsbereich (16) ausbildend, überlappend angeordnet sind, wobei die Fluidkammereinheit (9) zumindest teilweise, bevorzugt vollständig, innerhalb des zweiten Überlappungsbereichs (16) zwischen dem Abdeckelement (12) und dem Plattenelement (11) angeordnet ist, wobei das Abdeckelement (12) und das Plattenelement (11) zumindest teilweise, bevorzugt vollständig, innerhalb des ersten Überlappungsbereichs (14) zwischen dem Sitzplattenteil (3) und dem Sitzpolsterteil (4) angeordnet sind.

6. Fahrzeugsitz (1) nach Anspruch 4 oder 5,
**dadurch gekennzeichnet, dass**
das Abdeckelement (12) mechanisch mit der Fluidkammereinheit (9) verbunden ist, wobei das Abdeckelement (12) aus einem flexiblen Material, bevorzugt einem textilen Material, besteht, und wobei das Plattenelement (11) planar ausgebildet ist und aus einem flexiblen Material, bevorzugt einem Kunststoff, besteht.

7. System (100), bevorzugt angeordnet in einem Fahrzeug, umfassend einen Fahrzeugsitz (1) nach einem der vorhergehenden Ansprüche und eine zumindest signaltechnisch mit der Steuereinheit (22) verbundene Aktivierungseinheit (25), welche ausgebildet ist, ein Aktivierungssignal an die Steuereinheit (22) zu übermitteln, um ein Einbringen und/oder Entnehmen eines Fluids in/aus mindestens eine(r) Fluidkammer (10) von mindestes zwei Fluidkammern (10) einer Fluidkammereinheit (9) zu initiieren.

8. System (100) nach Anspruch 7,
**dadurch gekennzeichnet, dass**
die Aktivierungseinheit (25) eine manuell betätigbare Bedieneinheit (23) und/oder eine Körperfunktionserkennungseinheit umfasst, wobei die Körperfunktionserkennungseinheit dazu ausgebildet ist, mindestens eine Körperfunktion einer auf dem Fahrzeugsitz (1) sitzenden Person zu erfassen, und zumindest einen ersten Sensor aufweist.

9. Verfahren zum Betreiben eines Systems (100) nach Anspruch 7 oder 8, umfassend die Schritte:
a) Empfangen eines Aktivierungssignals ausgehend von einer Aktivierungseinheit (25) durch eine Steuereinheit (22);
b) Ausgeben eines pulsweitenmodulierten Steuersignals durch die Steuereinheit (22) an eine Fluidfördereinheit (21);
c) Einbringen eines Fluids in mindestens eine Fluidkammer (10) von mindestes zwei Fluidkammern (10) einer Fluidkammereinheit (9).

10. Fahrzeug, insbesondere Kraftfahrzeug, umfassend das System (100) nach einem der Ansprüche 7 oder 8.
